# EUROPEAN PATENT APPLICATION

(11) **EP 4 498 070 A1**
(43) Date of publication of application: **29.01.2025**
(21) Application number: 24154372.7
(22) Date of filing: 29.01.2024
(51) Int. Cl.: G01N 21/65, G01N 27/48, G01N 33/543, B82Y 10/00, B82Y 15/00, G01N 27/327

(54) **SURFACE-ENHANCED RAMAN SPECTROSCOPY SUBSTRATE, AND METHOD AND SYSTEM FOR DETECTING BIOMARKER BY USING SURFACE-ENHANCED RAMAN SPECTROSCOPY AND CYCLIC VOLTAMMETRY**

(30) Priority: 27.07.2023 KR 20230098228
(71) Applicant: Korea Advanced Institute of Science and Technology, Daejeon 34141 (KR)
(72) Inventor: JUNG, Yeonsik, Daejeon 34141 (KR); KIM, Minjoon, Daejeon 34141 (KR); LEE, Gyu Rac, Daejeon 34141 (KR); KU, Minjae, Daejeon 34141 (KR)
(74) Representative: Viering, Jentschura & Partner mbB Patent- und Rechtsanwälte

(57) **Abstract**

A surface-enhanced Raman spectroscopy substrate includes a multi-stage nanolattice structure including a first nanolattice including silver (Ag), a second nanolattice including gold (Au), and a third nanolattice including platinum (Pt). The surface-enhanced Raman spectroscopy substrate may be used for detecting different target materials.

## Description

### CROSS-REFERENCE TO RELATED APPLICATION

This application claims priority under 35 USC § 119 to Korean Patent Application No. 10-2023-0098228 filed on July 27, 2023 in the Korean Intellectual Property Office (KIPO), the entire disclosure of which is incorporated herein by reference.

### BACKGROUND

### 1. Field

Embodiments of the present disclosure relate to a detection system using surface-enhanced Raman spectroscopy. More particularly, embodiments of the present disclosure relate to a surface-enhanced Raman spectroscopy substrate, and a method and a system for detecting a biomarker by using surface-enhanced Raman spectroscopy and cyclic voltammetry.

### 2. Description of the Related Art

Biomarkers are biological indicators capable of detecting a variation in a body caused by occurrence of a disease or the like with molecular information including DNA, RNA, metabolites, proteins, and the like. The biomarkers may be used for diagnosis and prognosis observation of a disease, a customized medical technology, and development of an intelligent healthcare system for establishing a patient-friendly treatment environment. For example, biomarkers that are being clinically used for cancer diagnosis include prostate-specific antigen (PSA), carcinoembryonic antigen (CEA), and alpha-fetoprotein (AFP).

However, diagnosis using a single biomarker may have low accuracy. For example, according to meta-analysis, in a case of the PSA, a sensitivity for prostate cancer remains at about 70%, and a specificity for prostate cancer remains at 58%. In addition, one biomarker is frequently involved in a plurality of cancer types. Therefore, recently, a multi-biomarker panel-based diagnosis that simultaneously uses a plurality of biomarkers has been studied.

Multiplexing biosensors currently under development have a horizontal array-type sensor structure, and measure and quantify a plurality of biomarkers, respectively, which increases required clinical samples, measurement time, and cost. It is difficult to accurately quantify each of components due to false positive problems and mutual interference effects caused by non-specific binding of antibodies, aptamers, and the like used in a selective binding process of biomarkers.

### [Documents of Related Art]

### [Patent Documents]

1) Korean Unexamined Patent Publication No. 2021-0137215
2) Korean Unexamined Patent Publication No. 2014-0140179
3) Korean Patent Registration No. 1589039

### SUMMARY

One object of the present disclosure is to provide a surface-enhanced Raman spectroscopy substrate that is usable for multiple detection.

Another object of the present disclosure is to provide a detection method capable of improving accuracy of detection and quantification of a plurality of target materials.

Still another object of the present disclosure is to provide a detection system capable of improving accuracy of detection and quantification of a plurality of target materials.

According to an embodiment of the present disclosure, a surface-enhanced Raman spectroscopy substrate includes a multi-stage nanolattice structure including a first nanolattice including silver (Ag), a second nanolattice including gold (Au), and a third nanolattice including platinum (Pt).

In an embodiment, at least one of the first nanolattice, the second nanolattice, and the third nanolattice includes a first nanowire array including a plurality of nanowires extending in a first direction and a second nanowire array including a plurality of nanowires extending in a second direction intersecting the first direction. The the first nanowire array and the second nanowire array are welded in a contact region.

In an embodiment, the second nanolattice is disposed on the first nanolattice, and the third nanolattice is disposed on the second nanolattice.

In an embodiment, the second nanolattice has a pitch greater than a pitch of the first nanolattice, and the third nanolattice has a pitch greater than the pitch of the second nanolattice.

In an embodiment, each of the first to third nanolattices includes a plurality of nanowires. The nanowire of the first nanolattice and the nanowire of the second nanolattice are exposed through an opening of the third nanolattice.

In an embodiment, the first to third nanolattices have mutually different pitches.

In an embodiment, the first to third nanolattices have antibodies immobilized on surfaces of the first to third nanolattices, respectively.

In an embodiment, the antibodies immobilized on the first to third nanolattices are different from each other.

In an embodiment, the third nanolattice is heated at 500 °C to 850 °C.

According to an embodiment of the present disclosure, a method for detecting a biomarker includes allowing a sample including a biomarker to make contact with a surface-enhanced Raman spectroscopy substrate including a multi-stage nanolattice structure, identifying a biomarker bound to the surface-enhanced Raman spectroscopy substrate through Raman spectroscopy analysis, and quantifying the biomarker through cyclic voltammetry measurement of the multi-stage nanolattice structure.

In an embodiment, the method further includes binding an aptamer, which is bound to a Raman label, to the biomarker bound to the surface-enhanced Raman spectroscopy substrate before performing the Raman spectroscopy analysis.

In an embodiment, the multi-stage nanolattice structure includes at least three nanolattices including mutually different materials. The aptamer includes a first aptamer bound to rhodamine 6G (R6G), a second aptamer bound to methylene blue (MB), and a third aptamer bound to malachite green (MG).

In an embodiment, a concentration of the third aptamer is greater than each of a concentration of the first aptamer and a concentration of the second aptamer.

In an embodiment, the malachite green is bound to both a head and a tail of the third aptamer. The rhodamine 6G and the methylene blue are bound to one of a head or a tail of the first aptamer and the second aptamer.

In an embodiment, the multi-stage nanolattice structure includes a plurality of nanolattices including mutually different materials. The quantifying of the biomarker includes performing the cyclic voltammetry measurement of the multi-stage nanolattice structure, and calculating an amount of the biomarker based on a variation of a reduction voltage peak corresponding to the material of each of the nanolattices from a result obtained through the cyclic voltammetry measurement.

According to an embodiment of the present disclosure, a system for detecting a biomarker includes a surface-enhanced Raman spectroscopy substrate including a multi-stage nanolattice structure bindable with a plurality of biomarkers in a sample; a light source configured to emit a light to the surface-enhanced Raman spectroscopy substrate bound to the biomarker; an identifying part configured to identify the biomarker by analyzing Raman spectroscopy of the light reflected from the surface-enhanced Raman spectroscopy substrate; and a quantifying part configured to quantify the biomarker through cyclic voltammetry measurement of the surface-enhanced Raman spectroscopy substrate on which the biomarker is collected.

According to the embodiments, a target material may be rapidly detected by using surface-enhanced Raman spectroscopy.

In addition, a plurality of target materials may be simultaneously detected by using a multi-stage nanolattice structure that may be selectively bound to mutually different materials.

In addition, nanolattices may have mutually different pitches within a multi-stage nanolattice structure, so that a surface enhancement effect of a lower nanolattice may be prevented from deteriorating.

In addition, a surface enhancement effect may be increased through heating of Pt, which is known to have a low surface enhancement effect.

In addition, identification and quantification of a target material may be performed through a combination of surface-enhanced Raman spectroscopy and cyclic voltammetry.

In addition, multiple detection of biomarkers that are difficult to be identified and distinguished, such as proteins, may be performed by using an aptamer bound to a label.

In addition, accuracy of multiple detection may be improved through a combination of labels suitable for the multiple detection, and the accuracy of the multiple detection may be improved by varying the number of labels bound to the aptamer.

### BRIEF DESCRIPTION OF THE DRAWINGS

FIG. 1 is a cross-sectional view schematically showing a process of forming a nanowire array in a method for manufacturing a surface-enhanced Raman spectroscopy substrate according to one embodiment of the present disclosure.
FIG. 2 is a cross-sectional view schematically showing a process of transferring the nanowire array in the method for manufacturing the surface-enhanced Raman spectroscopy substrate according to one embodiment of the present disclosure.
FIG. 3 is a plan view showing a single layer of a nanolattice in the method for manufacturing the surface-enhanced Raman spectroscopy substrate according to one embodiment of the present disclosure.
FIG. 4 is an exploded perspective view showing a surface-enhanced Raman spectroscopy substrate according to one embodiment of the present disclosure.
FIG. 5 is a schematic view showing immobilizing of an antibody on the surface-enhanced Raman spectroscopy substrate according to one embodiment of the present disclosure.
FIG. 6 is a schematic view schematically showing a system for detecting a biomarker according to one embodiment of the present disclosure.
FIG. 7 is a schematic view for describing a method for detecting a biomarker according to one embodiment of the present disclosure.
FIG. 8 is a graph showing Raman spectroscopy obtained after dropping R6G (10⁻⁶ M) on a third nanolattice according to Example 1.
FIG. 9 shows a scanning electron microscope (SEM) photograph (a) after a second nanolattice is transferred onto a first nanolattice, and an SEM photograph (b) after the third nanolattice is transferred onto the second nanolattice according to Example 1.
FIG. 10 is a graph showing a result of cyclic voltammetry measurement of a multi-stage nanolattice structure according to Example 1.

### DETAILED DESCRIPTION OF THE EMBODIMENTS

Various embodiments will be described more fully hereinafter with reference to the accompanying drawings, in which some embodiments are shown. The invention may, however, be embodied in many different forms and should not be construed as limited to the embodiments set forth herein. Rather, these embodiments are provided so that this description will be thorough and complete, and will fully convey the scope of the invention to those skilled in the art. In the drawings, the sizes and relative sizes of layers and regions may be exaggerated for clarity.

It will be understood that when an element or layer is referred to as being "on," "connected to" or "coupled to" another element or layer, it can be directly on, connected or coupled to the other element or layer or intervening elements or layers may be present. In contrast, when an element is referred to as being "directly on," "directly connected to" or "directly coupled to" another element or layer, there are no intervening elements or layers present. Like numerals refer to like elements throughout. As used herein, the term "and/or" includes any and all combinations of one or more of the associated listed items.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the invention.

The terminology used herein is for the purpose of describing particular embodiments only and is not intended to be limiting of the invention. As used herein, the singular forms "a," "an" and "the" are intended to include a plurality of forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### Surface-enhanced Raman Spectroscopy Substrate and Method for Manufacturing the Same

According to one embodiment of the present disclosure, a surface-enhanced Raman spectroscopy substrate may be manufactured by using thermally assisted nanotransfer printing (T-nTP).

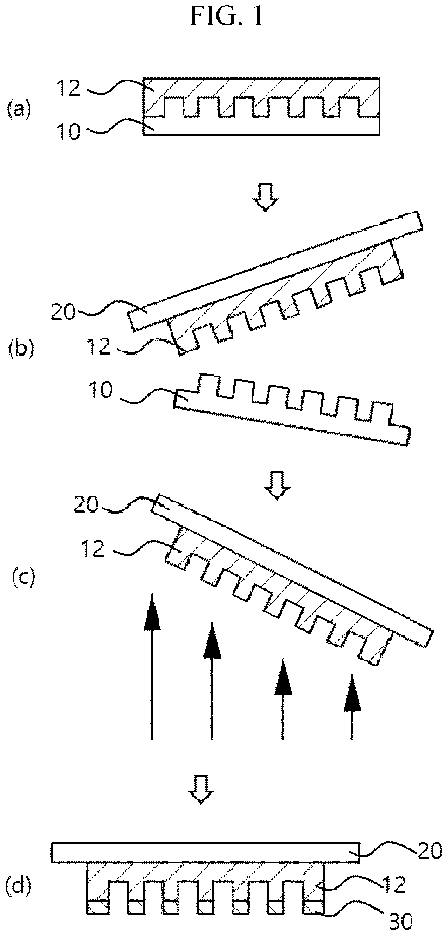

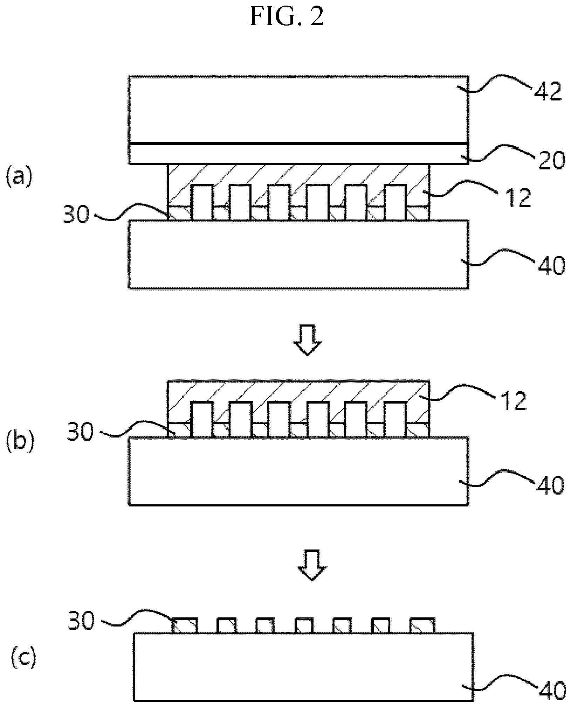

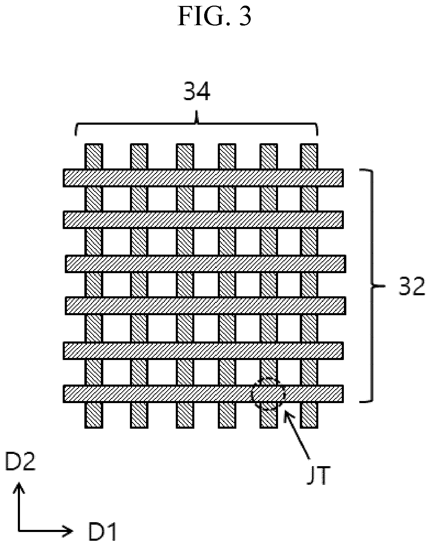

FIG. 1 is a cross-sectional view schematically showing a process of forming a nanowire array in a method for manufacturing a surface-enhanced Raman spectroscopy substrate according to one embodiment of the present disclosure. FIG. 2 is a cross-sectional view schematically showing a process of transferring the nanowire array in the method for manufacturing the surface-enhanced Raman spectroscopy substrate according to one embodiment of the present disclosure. FIG. 3 is a plan view showing a single layer of a nanolattice in the method for manufacturing the surface-enhanced Raman spectroscopy substrate according to one embodiment of the present disclosure.

Referring to (a) of FIG. 1, a master mold 10 having a surface on which a pattern is formed may be coated with a polymer to form a duplication mold 12. For example, a concavo-convex surface (the surface on which the pattern is formed) of the master mold 10 may be coated with a polymer composition including the polymer and a solvent by using spin coating, deep coating, or spray coating, and the solvent may be removed (dried), so that the duplication mold 12 may be formed. When viewed in a cross-sectional view, the concavo-convex surface of the master mold 10 may have a shape in which protrusions and recesses are repeatedly formed in a horizontal direction. In addition, a horizontal length of the protrusion and a horizontal length of the recess may be equal to or different from each other, and may be variously adjusted according to a shape and a pitch of the pattern to be transferred.

For example, the duplication mold 12 may include, but is not limited to, polymethyl methacrylate (PMMA), polystyrene, poly-4-viyl pyridine, or a combination thereof, and may be formed of various polymer materials. In addition, the duplication mold 12 may have a single-layer structure or a multilayer structure including mutually different materials.

In addition, self-assembled monolayer (SAM) coating using a PDMS brush polymer having low surface energy or a hydrophobic material such as hexamethylene disilazane (HMDS) may be performed on the surface of the master mold 10, on which the pattern is formed, in advance, so that the duplication mold 12 may be easily separated from the master mold 10 in the following step.

Referring to (b) of FIG. 1, the duplication mold 12 may be separated from the master mold 10. According to one embodiment, an adhesive film 20 may be attached to a top surface of the duplication mold 12 (a surface that is opposite to a surface of the duplication mold on which a pattern is transferred), and an external force may be applied, so that the duplication mold 12 and the master mold 10 may be physically separated from each other. An adhesive surface of the adhesive film 20 may be coated with an adhesive.

Since the duplication mold 12 is formed to fill a concavo-convex portion of the pattern of the surface of the master mold 10, the duplication mold 12 may have a concavo-convex pattern corresponding to an inverse shape of the pattern of the surface of the master mold 10.

Referring to (c) and (d) of FIG. 1, a functional material may be deposited on a partial region of the duplication mold 12 to form a nanowire array 30. According to one embodiment, while the duplication mold is tilted such that the surface of the duplication mold 12 on which the deposition is performed and a deposition direction may have a predetermined angle, when E-beam lithography, thermal evaporation, sputtering, or the like is performed, the functional material may be deposited only on the partial region including and surrounding a protruding portion (edge) of the pattern of the surface of the duplication mold 12. Accordingly, a pattern of the functional material may be obtained without a photolithography process. For example, when the pattern of the surface of the duplication mold 12 has a wire array shape (when the protrusion or the recess has a shape extending in one direction), the nanowire array 30 may be formed through the inclined deposition.

The functional material may include a metal such as Au, Ag, Cu, Ni, Pt, Cr, Co, or Pd, but embodiments of the present disclosure are not limited thereto, and various materials may be used as the functional material as necessary.

Next, the nanowire array 30 may be transferred to a transfer substrate.

Referring to (a) and (b) of FIG. 2, the nanowire array 30 may be brought into contact with a surface of the transfer substrate 40, and the adhesive film 20 may be pressed and heated by using a pressing member 42. Accordingly, bonding strength between the adhesive film 20 and the duplication mold 12 may be weakened by the heat, so that the adhesive film 20 may be easily removed from the duplication mold 12. A heating temperature may be greater than or equal to 100 °C, for example, 100 °C to 300 °C, but the embodiments of the present disclosure are not limited thereto.

The duplication mold 12 remaining on the nanowire array 30 may be removed by using a solvent. For example, in order to remove the duplication mold 12, an organic solvent such as toluene, acetone, or isopropyl alcohol may be provided to the duplication mold 12 to remove the duplication mold 12.

As a result, as shown in (c) of FIG. 2, the nanowire array 30 may be transferred onto the transfer substrate 40. For example, a line width of the nanowire array 30 may be 10 nm to 100 nm, and a pitch of the nanowire array 30 may be 20 nm to 500 nm, but the line width and the pitch are not limited thereto.

According to one embodiment, the above process may be repeatedly performed to form a nanolattice. For example, referring to FIG. 3, a first nanowire array 32 including a plurality of nanowires extending in a first direction D1 may be formed on the transfer substrate. The process shown in FIG. 1 may be repeatedly performed to form the nanowire array on the duplication mold, heat-assisted transfer may be performed in a method shown in FIG. 2 on the transfer substrate 40 onto which the first nanowire array 32 is transferred such that the first nanowire array 32 and an additional nanowire array may intersect each other, so that a nanolattice including a second nanowire array 34 may be formed. For example, the second nanowire array 34 may include a plurality of nanowires extending in a second direction D2 perpendicular to the first direction D1.

An opening of the nanolattice may be defined between adjacent nanowires of the first nanowire array 32 and between adjacent nanowires of the second nanowire array 34.

Since the heat is provided in a process of binding the first nanowire array 32 to the second nanowire array 34, a contact region JT (intersection region) of the first nanowire array 32 and the second nanowire array 34 may be welded. Accordingly, stability of the nanolattice may be improved, and the nanolattice may be prevented from being deformed or damaged in subsequent processes.

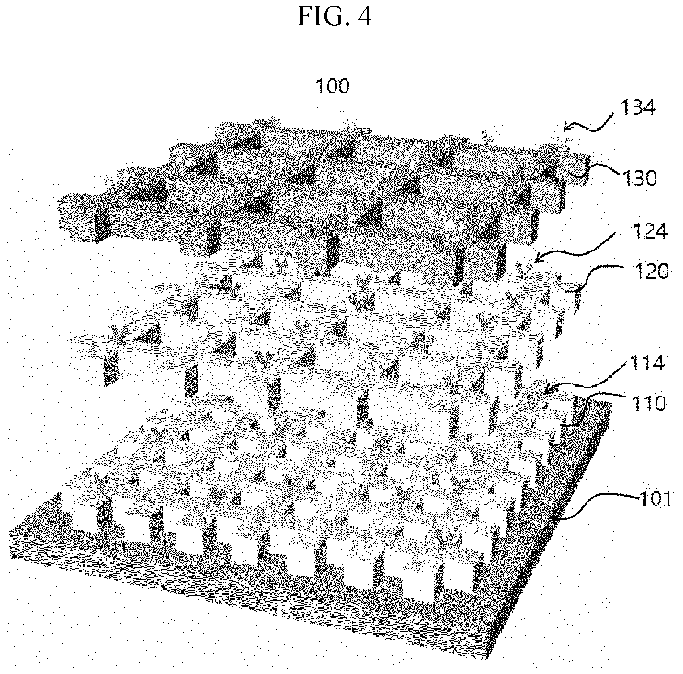

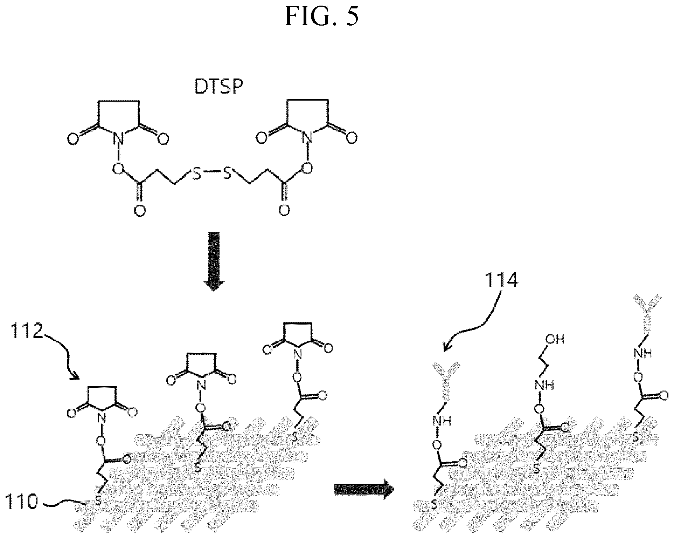

FIG. 4 is an exploded perspective view showing a surface-enhanced Raman spectroscopy substrate according to one embodiment of the present disclosure. FIG. 5 is a schematic view showing immobilizing of an antibody on the surface-enhanced Raman spectroscopy substrate according to one embodiment of the present disclosure.

Referring to FIG. 4, a surface-enhanced Raman spectroscopy substrate 100 may include a multi-stage nanolattice structure.

For example, the multi-stage nanolattice structure may include a first nanolattice 110, a second nanolattice 120 disposed on the first nanolattice 110, and a third nanolattice 130 disposed on the second nanolattice 120. The multi-stage nanolattice structure may be disposed on a base substrate 101. The base substrate 101 may be conductive, or may include an electrode (conductive terminal) electrically connected to the multi-stage nanolattice structure.

According to one embodiment, the nanolattices of the multi-stage nanolattice structure may include mutually different materials. For example, the first nanolattice 110 may include Ag, the second nanolattice 120 may include Au, and the third nanolattice 130 may include Pt.

According to one embodiment, the third nanolattice 130 including Pt may be heated. The heated Pt may have increased crystallinity, which may improve a surface enhancement effect. Accordingly, the surface enhancement effect of Pt, which is lower than a surface enhancement effect of each of Ag and Au, may be improved so as to be used as a material for the surface-enhanced Raman spectroscopy substrate.

For example, a heating temperature for Pt may be 500 °C to 850 °C, and preferably 650 °C to 800 °C. When the heating temperature is excessively high, the crystallinity may be reduced by melting so that the surface enhancement effect may not be increased.

In addition, the nanolattices may have mutually different pitches. According to one embodiment, the first nanolattice 110 may have a first pitch (hereinafter, the pitch may refer to a pitch between adjacent nanowires), the second nanolattice 120 may have a second pitch that is greater than the first pitch, and the third nanolattice 130 may have a third pitch that is greater than the second pitch. In addition, a size (an area when viewed in plan view) of an opening (a unit opening) of the second nanolattice 120 may be greater than a size of an opening of the first nanolattice 110, and a size of an opening of the third nanolattice 130 may be greater than the size of the opening of the second nanolattice 120. According to one embodiment, the nanowire of the second nanolattice 120 and the nanowire of the first nanolattice 110 may be exposed through the opening of the third nanolattice 130. In other words, when viewed in a plan view the opening of the third nanolattice 130 may overlap the nanowire of the second nanolattice 120 and the nanowire of the first nanolattice 110.

According to one embodiment, since the size of the opening the upper nanolattice is greater than the size of the opening of the lower nanolattice in the multi-stage nanolattice structure, the lower nanolattice may be easily exposed to a target material coming from a top of the multi-stage nanolattice structure. In addition, a deviation between areas of the nanolattices exposed to the sample may be reduced to improve reliability of detection.

In addition, in consideration of a difference between surface enhancement effects of the materials of the nanolattices (Ag > Au > Pt), a nanolattice formed of a material having a relatively large surface enhancement effect may be disposed in a lower portion (a region in which the surface enhancement effect is reduced by an upper layer), and a nanolattice formed of a material having a relatively small surface enhancement effect may be disposed in an upper portion, so that a decrease in the reliability of the detection caused by the difference between the surface enhancement effects may be prevented.

Each of the nanolattices of the multi-stage nanolattice structure may be formed through the method described with reference to FIGS. 1 to 3. When the transfer substrate of the nanolattice is removed through an etchant or the like, and the nanolattice is brought into contact with another nanolattice, the nanolattices may be bound to each other by a van der Waals' force or the like without a separate adhesive, so that the multi-stage nanolattice structure may be formed.

The surface-enhanced Raman spectroscopy substrate may be used to detect a biomarker, and more particularly, the surface-enhanced Raman spectroscopy substrate may be effectively used for multiple detection of biomarkers. For the multiple detection, the nanolattices may be designed to be selectively bound to mutually different biomarkers, respectively.

According to one embodiment, the first nanolattice 110 may include a first antibody 114, the second nanolattice 120 may include a second antibody 124, and the third nanolattice 130 may include a third antibody 134. The antibodies may be bound to mutually different target materials (biomarkers) through antigen-antibody binding, respectively. In addition, after the antibody collects the target material, the target materials may be bound to aptamers connected to mutually different Raman labels according to types of the target materials, respectively.

According to one embodiment, in order to bind the antibody to the nanolattice, a linker such as dithiobis-N-succinimidyl propionate (DTSP) may be used.

For example, as shown in FIG. 5, the DTSP may be provided to the nanolattice 110 to form a linker 112 on a surface of the nanolattice, and the antibody 114 may be bound to the linker.

Immobilizing of an antibody on each of the nanolattices may be performed after each of the nanolattices is formed, and subsequently, the nanolattices may be bound to each other to form the multi-stage nanolattice structure. In addition, the heating of the Pt nanolattice may be performed after the nanolattice including Pt is formed, and subsequently, the antibody may be immobilized on the Pt nanolattice.

In addition, since the nanolattices are formed of mutually different materials according to the embodiments of the present disclosure, when cyclic voltammetry measurement is performed, reduction voltage peaks having mutually different values may be observed by the nanolattices. Characteristics (a current value, an area, etc.) of the reduction voltage peak may vary according to an amount of target materials bound to each of the nanolattices. Accordingly, after the substrate reacts with the sample, each of the bound target materials or the target materials included in the sample may be quantified based on a variation in a reduction voltage peak (before/after the reaction) obtained through the cyclic voltammetry measurement.

### Method and System for Detecting Biomarker

According to one embodiment of the present disclosure, a method for detecting a biomarker may include: allowing a sample including a biomarker to make contact with a surface-enhanced Raman spectroscopy substrate including a multi-stage nanolattice structure; identifying a biomarker bound to the surface-enhanced Raman spectroscopy substrate through Raman spectroscopy analysis; and quantifying the biomarker through cyclic voltammetry measurement of the multi-stage nanolattice structure.

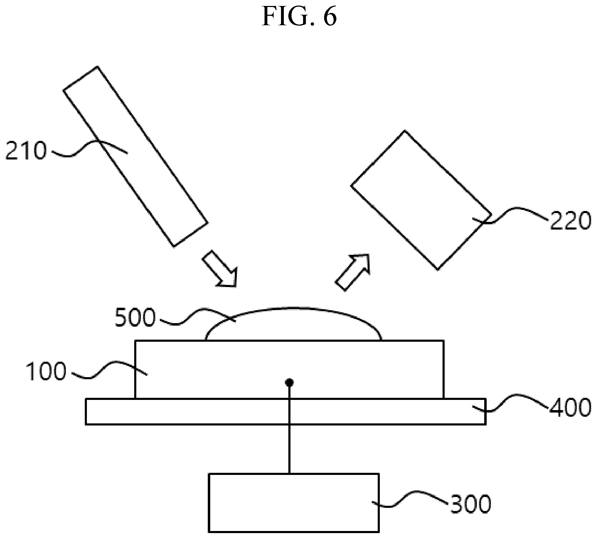

FIG. 6 is a schematic view schematically showing a system for detecting a biomarker according to one embodiment of the present disclosure.

Referring to FIG. 6, according to one embodiment of the present disclosure, a system for detecting a biomarker may include: a surface-enhanced Raman spectroscopy substrate 100 bindable with a biomarker in a sample 500; a light source 210 configured to emit a light to the surface-enhanced Raman spectroscopy substrate bound to the biomarker; an identifying part 220 configured to identify the biomarker by analyzing Raman spectroscopy of the light reflected from the surface-enhanced Raman spectroscopy substrate; and a quantifying part 300 configured to quantify the biomarker through cyclic voltammetry measurement of the surface-enhanced Raman spectroscopy substrate on which the biomarker is collected. In addition, the system may further include a stage 400 configured to support the surface-enhanced Raman spectroscopy substrate 100.

The surface-enhanced Raman spectroscopy substrate may be identical to the surface-enhanced Raman spectroscopy substrate described above.

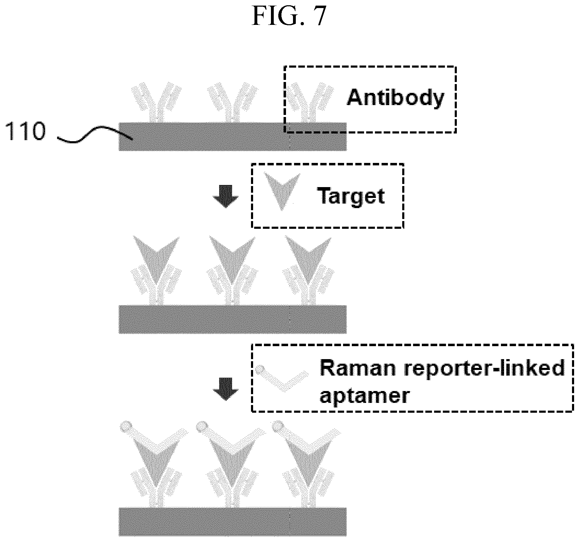

FIG. 7 is a schematic view for describing a method for detecting a biomarker according to one embodiment of the present disclosure.

Referring to FIG. 7, a sample may be brought into contact with a nanolattice 110 of a multi-stage nanolattice structure having an antibody bound to a surface of the multi-stage nanolattice structure, so that a biomarker Target in the sample may be bound to the antibody.

For example, an appropriate amount of sample may be dropped onto the surface-enhanced Raman spectroscopy substrate, and contact may be maintained for a sufficient time for the biomarker in the sample to be bound to the antibody. In addition, the substrate may be moved or rotated so that the antibody and the biomarker may be easily bound to each other.

The biomarker may be derived from a biological organism. For example, the sample may include a biological fluid. The biological fluid may include urine, blood, plasma, serum, saliva, semen, excrement, sputum, a cerebrospinal fluid, a tear, mucus, an amniotic fluid, and the like. In addition, the sample may include a biological tissue of a humans, an animal, a plant, a bacterium, a mycete, and a virus, a cell thereof, or an intracellular material thereof. In addition, the sample may include an environmental sample such as drinking water or food.

According to one embodiment, after the antibody and the biomarker are bound to each other, an aptamer bound to a Raman label may be bound to the biomarker. When the biomarker is a protein, detection of a peak distinguished through Raman spectroscopy analysis may not be easily performed. According to one embodiment of the present disclosure, mutually different Raman labels may be bound according to a type of the biomarker, and a peak corresponding to the Raman label may be determined, so that reliability of multiple detection may be improved.

When a plurality of biomarkers are to be detected, mutually different aptamers that may be selectively bound to the biomarkers, respectively, may be used, and the aptamers may be bound to mutually different Raman labels, respectively.

For example, the following Table 1 shows biomarkers used in diagnosis of lung cancer. Among the following biomarkers, there is a study showing that diagnosis accuracy is high (with a sensitivity of 75% and a specificity of 82%) when the diagnosis is performed through a combination of carcinoembryonic antigen (CEA), CYFRA 21-1, and neuron-specific enolase (NSE).

**Table 1**

| **Cancer** | **Biomarker** |
|---|---|
| Lung cancer | ✔ **Carcinoembryonic Antigen (CEA)** |
| | Retinol-binding protein (RBP) |
| | α1-antitrypsin (A1AT) |
| | squamous cell carcinoma antigen |
| | CA-125 |
| | α-Fetoprotein (AFP) |
| | ✔ **CYFRA 21-1** |
| | chromogranin A |
| | ✔ **neuron-specific enolase (NSE)** |

A structure of an aptamer corresponding to (bindable to) the biomarker has been known as follows.

| | |
|---|---|
| **CEA aptamer** | DNA aptamer : 5'-ATACCAGCTTATTCAATT-3' |
| | Ref: J. Mater. Chem. B, 2017, 5, 1594-1600 |
| **CYFRA 21-1 aptamer** | DNA aptamer : 5'-CGCCCCTGACACCATTCCTCCCTTC-3' |
| | Ref: https://doi.org/10.1016/j.chemosphere.2022.134636 |
| **Neuron-specific enolase (NSE)** | DNA aptamer : 5'-TCACACACGGACCTCTCCTACATTAATTGCGCATTTCGTT-3' |
| | Ref: **https://pubs.rsc.org/en/content/articlepdf/2079/re/c9ra00785g** |
| | **:** RSC Adv., 2019, 9, 15513 |

For example, when the above three biomarkers are to be simultaneously detected from a sample (blood of a test subject, etc.), a first aptamer that may be selectively bound to the CEA, a second aptamer that may be selectively bound to the CYFRA 21-1, and a third aptamer that may be selectively bound to the NSE may be provided to the substrate so as to be bound to the corresponding biomarkers.

The first to third aptamers may be bound to mutually different labels. For example, the labels may be bound to a head and/or a tail of the aptamers.

Since the labels are for distinguishing the biomarkers from each other, it may be preferable that Raman peaks by the labels are easily distinguished from each other, and it may be preferable that Raman cross-sections are similar to each other in order to identify each of the Raman peaks.

According to one embodiment, a combination of a plurality of labels (dyes) to be bound to the first to third aptamers, respectively, may include rhodamine 6G (R6G), methylene blue (MB), and malachite green (MG). The label-aptamer combination does not have to be specified, while the same label has to be bound to the same aptamers. For example, the first aptamer may be bound to the R6G, the second aptamer may be bound to the MB, and the third aptamer may be bound to the MG.

According to one embodiment, a first nanolattice including Ag may be bound to a CYFRA 21-1-specific antibody, in which the aptamer bound to the MB may be used to label the CYFRA 21-1, a second nanolattice including Au may be bound to an NSE-specific antibody, in which the aptamer bound to the R6G may be used to label the NSE, and a third nanolattice including Pt may be bound to a CEA-specific antibody, in which the aptamer bound to the MG may be used to label the CEA.

In order to increase accuracy of the Raman spectroscopy analysis, it may be preferable that a difference between intensities of the Raman peaks of the labels is not large. Therefore, the following method may be considered so that the intensity of the Raman peak of each of the labels is located within a predetermined range.

According to one embodiment, when the aptamers are provided to the substrate, concentrations of the aptamers may be different from each other. For example, when a Raman peak of the MG is weak, a concentration of the third aptamer bound to the MG may be greater than a concentration of each of the first aptamer and the second aptamer.

According to another embodiment, the numbers of the labels bound to the aptamers may be different from each other. For example, when the Raman peak of the MG is weak, the MG may be bound to both the head and the tail of the third aptamer, and the label may be bound to one of the head or the tail of the first aptamer and the second aptamer.

The quantifying part 300 may perform the cyclic voltammetry measurement of the surface-enhanced Raman spectroscopy substrate bound to the biomarker to quantify the biomarker based on the cyclic voltammetry measurement.

According to the embodiments of the present disclosure, the surface-enhanced Raman spectroscopy substrate may include a plurality of nanolattices including mutually different materials. Therefore, when the cyclic voltammetry measurement of the surface-enhanced Raman spectroscopy substrate is performed, reduction voltage peaks having mutually different values may be observed by the nanolattices. Characteristics (a current value, an area, etc.) of the reduction voltage peak may vary according to an amount of biomarkers bound to each of the nanolattices. Accordingly, after the substrate reacts with the sample to bind the substrate to the biomarker, the amount of the biomarkers may be calculated based on a variation in a reduction voltage peak (before/after the reaction) obtained through the cyclic voltammetry measurement. Accordingly, the biomarkers bound to the substrate or the biomarkers included in the sample may be distinguished and quantified. In addition, various known calibrations may be used for the quantification based on the cyclic voltammetry measurement.

According to the embodiments of the present disclosure, a target material may be rapidly detected by using surface-enhanced Raman spectroscopy.

In addition, a plurality of target materials may be simultaneously detected by using a multi-stage nanolattice structure that may be selectively bound to mutually different materials.

In addition, nanolattices may have mutually different pitches within a multi-stage nanolattice structure, so that a surface enhancement effect of a lower nanolattice may be prevented from deteriorating.

In addition, a surface enhancement effect may be increased through heating of Pt, which is known to have a low surface enhancement effect.

In addition, identification and quantification of a target material may be performed through a combination of surface-enhanced Raman spectroscopy and cyclic voltammetry.

In addition, multiple detection of biomarkers that are difficult to be identified and distinguished, such as proteins, may be performed by using an aptamer bound to a label.

In addition, accuracy of multiple detection may be improved through a combination of labels suitable for the multiple detection, and the accuracy of the multiple detection may be improved by varying the number of labels bound to the aptamer.

Hereinafter, effects of the present disclosure will be reviewed through specific examples and experiments.

### Example 1

### Preparation of First Nanolattice

A concavo-convex surface of a master mold having a pitch of 80 nm (where a protrusion width is 40 nm, and a recess width is 40 nm) was coated with PMMA, and dried to form a duplication mold. After an adhesive film is attached to a top surface of the duplication mold, the duplication mold was separated from the master mold.

While the duplication mold is tilted, deposition was performed to form a first Ag nanowire array. The first Ag nanowire array was placed on a SiO₂ wafer substrate, heated (150 °C), and pressed, and the adhesive film was removed. After the adhesive film is removed, isopropyl alcohol was applied to remove the duplication mold, and the first Ag nanowire array was transferred to the SiO₂ wafer substrate.

A second Ag nanowire array was formed in the same way as the first Ag nanowire array, and brought into contact with the first Ag nanowire array to intersect the first Ag nanowire array when viewed in a plan view. Next, in the same way as above, heating-pressing was performed, an adhesive film was removed, and a duplication mold was removed, so that a first nanolattice including the first Ag nanowire array and the second Ag nanowire array, which are welded to each other in a contact region, was prepared.

In order to immobilize an antibody on the first nanolattice, a dimethyl sulfoxide (DMSO) solution (NaBH₄ 75 mM) in which DTSP (3 mg/mL) is dissolved was incubated for 30 minutes, the first nanolattice was immersed in the solution for 30 minutes, and the first nanolattice was washed with DMSO and deionized water.

Next, the first nanolattice was immersed in a PBS solution (1 to 2 g/ml) including a CYFRA21-1-specific antibody (monoclonal antibody) for 4 hours, immersed in a PBS solution including 10% ethanolamine for 4 hours, and washed with 0.1M Tris-HCl (pH 8.0).

### Preparation of Second Nanolattice

A concavo-convex surface of a master mold having a pitch of 100 nm (where a protrusion width is 50 nm, and a recess width is 50 nm) was coated with PMMA, and dried to form a duplication mold. After an adhesive film is attached to a top surface of the duplication mold, the duplication mold was separated from the master mold.

While the duplication mold is tilted, deposition was performed to form a first Au nanowire array. The first Au nanowire array was placed on a SiO₂ wafer substrate, heated (150 °C), and pressed, and the adhesive film was removed. After the adhesive film is removed, isopropyl alcohol was applied to remove the duplication mold, and the first Au nanowire array was transferred to the SiO₂ wafer substrate.

A second Au nanowire array was formed in the same way as the first Au nanowire array, and brought into contact with the first Au nanowire array to intersect the first Au nanowire array when viewed in a plan view. Next, in the same way as above, heating-pressing was performed, an adhesive film was removed, and a duplication mold was removed, so that a second nanolattice including the first Au nanowire array and the second Au nanowire array, which are welded to each other in a contact region, was prepared.

In order to immobilize an antibody on the second nanolattice, a DMSO solution (NaBH₄ 75 mM) in which DTSP (3 mg/mL) is dissolved was incubated for 30 minutes, the second nanolattice was immersed in the solution for 30 minutes, and the second nanolattice was washed with DMSO and deionized water.

Next, the second nanolattice was immersed in a PBS solution (1 to 2 g/ml) including an NSE-specific antibody for 4 hours, immersed in a PBS solution including 10% ethanolamine for 4 hours, and washed with 0.1M Tris-HCl (pH 8.0).

### Preparation of Third Nanolattice

A concavo-convex surface of a master mold having a pitch of 200 nm (where a protrusion width is 50 nm, and a recess width is 150 nm) was coated with PMMA, and dried to form a duplication mold. After an adhesive film is attached to a top surface of the duplication mold, the duplication mold was separated from the master mold.

While the duplication mold is tilted, deposition was performed to form a first Pt nanowire array. The first Pt nanowire array was placed on a SiO₂ wafer substrate, heated (150 °C), and pressed, and the adhesive film was removed. After the adhesive film is removed, isopropyl alcohol was applied to remove the duplication mold, and the first Pt nanowire array was transferred to the SiO₂ wafer substrate.

A second Pt nanowire array was formed in the same way as the first Pt nanowire array, and brought into contact with the first Pt nanowire array to intersect the first Pt nanowire array when viewed in a plan view. Next, in the same way as above, heating-pressing was performed, an adhesive film was removed, and a duplication mold was removed, so that a third nanolattice including the first Pt nanowire array and the second Pt nanowire array, which are welded to each other in a contact region, was prepared.

Next, the third nanolattice was heated by performing heating in an Ar atmosphere for 1 minute or more.

Next, in order to immobilize an antibody on the third nanolattice, a DMSO solution (NaBH₄ 75 mM) in which DTSP (3 mg/mL) is dissolved was incubated for 30 minutes, the third nanolattice was immersed in the solution for 30 minutes, and the third nanolattice was washed with DMSO and deionized water.

Next, the third nanolattice was immersed in a PBS solution (1 to 2 g/ml) including a CEA-specific antibody for 4 hours, immersed in a PBS solution including 10% ethanolamine for 4 hours, and washed with 0.1M Tris-HCl (pH 8.0).

After antibody immobilization reactions of the first to third nanolattices, anti-IgG in which FITC, which is a fluorescent substance, is labeled was provided, and fluorescence was checked to confirm that the antibody was immobilized on each of the nanolattices.

### Preparation of Multi-stage Nanolattice Structure

After the SiO2 wafer substrate is removed from the second nanolattice by using an etchant (BOE), the separated second nanolattice was floated on purified water, and water transfer was performed on the first nanolattice.

After the SiO₂ wafer substrate is removed from the third nanolattice by using an etchant (BOE), the separated third nanolattice was floated on purified water, and water transfer was performed on the second nanolattice disposed on the first nanolattice.

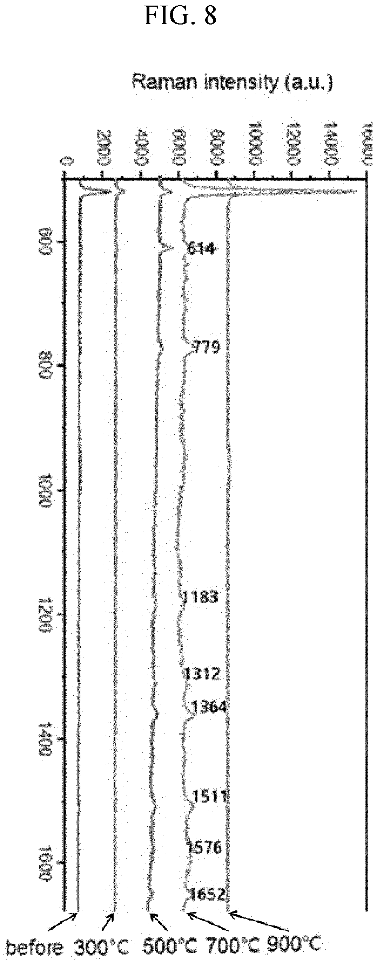

FIG. 8 is a graph showing Raman spectroscopy obtained after dropping R6G (10⁻⁶ M) on a third nanolattice according to Example 1.

Referring to FIG. 8, it was found that a surface enhancement effect is increased at a heating temperature of 500 °C or more, and it was found that the surface enhancement effect is increased most significantly at 700 °C, while the surface enhancement effect is decreased at 900 °C.

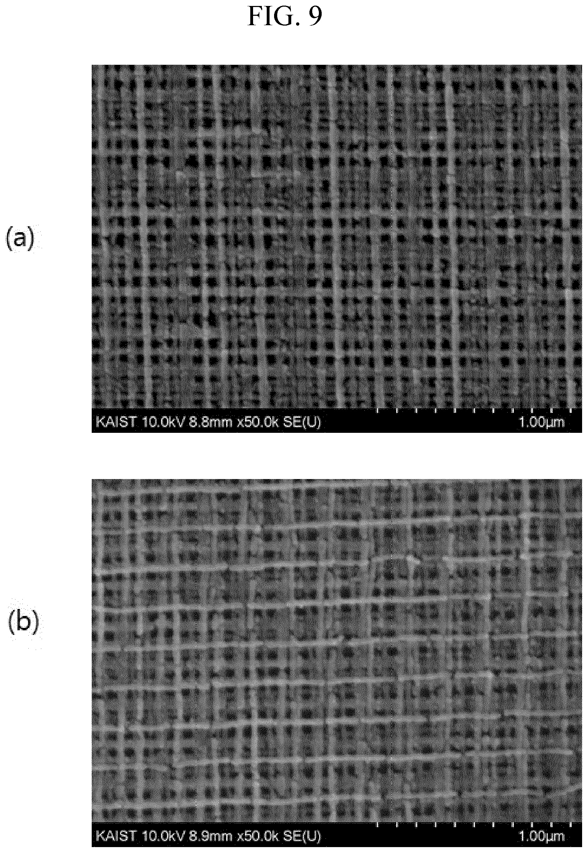

FIG. 9 shows a scanning electron microscope (SEM) photograph (a) after a second nanolattice is transferred onto a first nanolattice, and an SEM photograph (b) after the third nanolattice is transferred onto the second nanolattice according to Example 1.

Referring to FIG. 9, it was found that a lower nanolattice may be exposed by varying the pitch of each of the nanolattices.

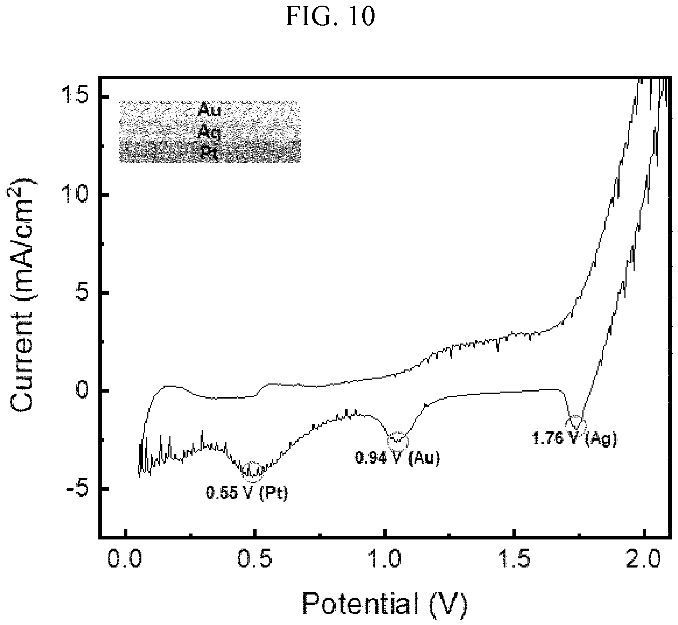

FIG. 10 is a graph showing a result of cyclic voltammetry measurement of a multi-stage nanolattice structure according to Example 1.

Referring to FIG. 10, it was found that reduction voltage peaks corresponding to the nanolattices (Ag, Au, and Pt) are distinguished and observed. Therefore, it may be understood that a plurality of biomarkers that are subjected to multiple detection may be quantified by performing cyclic voltammetry measurement of the multi-stage nanolattice structure.

Embodiments of the present disclosure may be used for detecting various materials, which can be detecded through surface-enhanced Raman spectroscopy analysis, including a biomarker.

The foregoing is illustrative of embodiments and is not to be construed as limiting thereof. Although a few embodiments have been described, those skilled in the art will readily appreciate that many modifications are possible in the embodiments without materially departing from the novel teachings and advantages of the present inventive concept. Accordingly, all such modifications are intended to be included within the scope of the present inventive concept as defined in the claims. Therefore, it is to be understood that the foregoing is illustrative of various embodiments and is not to be construed as limited to the specific embodiments disclosed, and that modifications to the disclosed embodiments, as well as other embodiments, are intended to be included within the scope of the appended claims.

## Claims

1. A surface-enhanced Raman spectroscopy substrate comprising:
a multi-stage nanolattice structure including a first nanolattice including silver (Ag), a second nanolattice including gold (Au), and a third nanolattice including platinum (Pt).

2. The surface-enhanced Raman spectroscopy substrate of claim 1, wherein at least one of the first nanolattice, the second nanolattice, and the third nanolattice includes:
a first nanowire array including a plurality of nanowires extending in a first direction; and
a second nanowire array including a plurality of nanowires extending in a second direction intersecting the first direction, and
the first nanowire array and the second nanowire array are welded in a contact region.

3. The surface-enhanced Raman spectroscopy substrate of claim 1, wherein the second nanolattice is disposed on the first nanolattice, and
the third nanolattice is disposed on the second nanolattice.

4. The surface-enhanced Raman spectroscopy substrate of claim 3, wherein the second nanolattice has a pitch greater than a pitch of the first nanolattice, and
the third nanolattice has a pitch greater than the pitch of the second nanolattice.

5. The surface-enhanced Raman spectroscopy substrate of claim 3, wherein each of the first to third nanolattices includes a plurality of nanowires, and
the nanowire of the first nanolattice and the nanowire of the second nanolattice are exposed through an opening of the third nanolattice.

6. The surface-enhanced Raman spectroscopy substrate of claim 1, wherein the first to third nanolattices have mutually different pitches.

7. The surface-enhanced Raman spectroscopy substrate of claim 1, wherein the third nanolattice is heated at 500 °C to 850 °C.

8. A method for detecting a biomarker, the method comprising:
allowing a sample including a biomarker to make contact with a surface-enhanced Raman spectroscopy substrate including a multi-stage nanolattice structure;
identifying a biomarker bound to the surface-enhanced Raman spectroscopy substrate through Raman spectroscopy analysis; and
quantifying the biomarker through cyclic voltammetry measurement of the multi-stage nanolattice structure.

9. The method of claim 8, wherein the multi-stage nanolattice structure includes a first nanolattice including silver (Ag), a second nanolattice including gold (Au), and a third nanolattice including platinum (Pt).

10. The method of claim 9, wherein the first to third nanolattices have mutually different antibodies immobilized on surfaces of the first to third nanolattices, respectively.

11. The method of claim 8, further comprising binding an aptamer, which is bound to a Raman label, to the biomarker bound to the surface-enhanced Raman spectroscopy substrate before performing the Raman spectroscopy analysis.

12. The method of claim 11, wherein the multi-stage nanolattice structure includes at least three nanolattices including mutually different materials, and
the aptamer includes a first aptamer bound to rhodamine 6G (R6G), a second aptamer bound to methylene blue (MB), and a third aptamer bound to malachite green (MG).

13. The method of claim 12, wherein a concentration of the third aptamer is greater than each of a concentration of the first aptamer and a concentration of the second aptamer.

14. The method of claim 12, wherein the malachite green is bound to both a head and a tail of the third aptamer, and
the rhodamine 6G and the methylene blue are bound to one of a head or a tail of the first aptamer and the second aptamer.

15. The method of claim 8, wherein the multi-stage nanolattice structure includes a plurality of nanolattices including mutually different materials, and
the quantifying of the biomarker includes:
performing the cyclic voltammetry measurement of the multi-stage nanolattice structure; and
calculating an amount of the biomarker based on a variation of a reduction voltage peak corresponding to the material of each of the nanolattices from a result obtained through the cyclic voltammetry measurement.
